# EUROPEAN PATENT SPECIFICATION

(11) **EP 3 793 709 B1**
(45) Date of publication and mention of the grant of the patent: **10.07.2024**
(21) Application number: 18725817.3
(22) Date of filing: 16.05.2018
(51) Int. Cl.: B01D 46/00

(54) **AIR FILTER CLEANER PROCESSING**
LUFTFILTERREINIGERVERARBEITUNG
TRAITEMENT D'UN DISPOSITIF DE NETTOYAGE DE FILTRE À AIR

(43) Date of publication of application: 24.03.2021
(73) Proprietor: ELECTROLUX APPLIANCES AKTIEBOLAG, 105 45 Stockholm (SE)
(72) Inventor: TANYILDIZ, Baris, Hangzhou, Zheijiang 310018 (CN)
(74) Representative: Electrolux Group Patents
(86) International application number: PCT/EP2018/062760
(87) International publication number: WO 2019/219187

(56) References cited:
- CN-U- 204 619 553
- JP-A- 2015 197 224
- JP-A- S63 175 613
- JP-U- S48 108 268
- US-A1- 2006 096 459
- US-A1- 2009 205 498

## Description

### TECHNICAL FIELD

The invention relates to cleaning of air-filters in air purifiers. In particular the present invention relates to an air purifier comprising a filter cleaner module.

### BACKGROUND

Air purifiers are used to clean air from undesired particles such as pollen, germs, virus, allergens, micro particles, dust etc. Typically, the air purification is performed by letting air flow through an air-filter.

Different types of air-purifiers exist. For example, so-called tower air purifiers can be used. In a tower air purifier, air can be passed from the sides of the tower air purifier, made to pass a filter and let out in the top of the tower air purifier. Other types of air purifiers exist.

The filter in the air purifier needs to be changed or cleaned from time to time to be efficient. Different methods for and devices for cleaning the filter of an air purifier are used. For tower air-purifiers, the filter is typically exchanged when the filter is no longer efficient.

There is a constant desire to improve air purifier. Hence, there exists a need for an improved air purifier and an improved mechanism for keeping the air-filters of air purifiers clean an efficient.

In US2009/205498A1 an air cleaning apparatus is disclosed including a housing defining an airflow passage, a blower retained by the housing and adapted when energized to move air through the airflow passage, a filter element disposed in the airflow passage and having an inlet surface for collecting particulate matter from the airflow. A nozzle element is adapted to engage the inlet surface and is connectable to a vacuum system for allowing the vacuum system to remove the particulate matter collected on the inlet surface. An emitter bathes the inlet surface with radiation to kill germs and detoxify impurities which collect thereon.

US2006/096459A1 discloses an air conditioner having an interior casing in which an air filter is provided, a filter cleaning part, a cleaning time drive member for moving the air filter and the filter cleaning part with respect to each other, and an ultraviolet light irradiation unit which irradiates the air filter with ultraviolet rays. The air filter and the filter cleaning part are relatively moved by the cleaning time drive member to remove dust adhered to the air filter, and the ultraviolet light irradiation unit irradiates an uncleaned face of the air filter with the ultraviolet rays.

### SUMMARY

It is an object of the present invention to provide an air purifier with an improved air filter cleaning mechanism. In particular, it is an object to provide an air purifier comprising :
- a casing and cylindrical filter, wherein the casing of the air purifier is polygonal such that a space is formed between a corner of the polygonal casing and the cylindrical filter, the air purifier further comprising
- a filter cleaner module located in said space for cleaning and sterilizing an air-filter, the filter cleaner module being configured to:
   - perform mechanical cleaning of the air-filter by making a cleaning member contact the surface of the air-filter for a first period of time, and then
   - perform sterilization of the air-filter by applying UV light from at least one UV light source located in the filter cleaning module.

This object and or others are obtained by an air purifier as set out in the appended claims.

As has been realized by the inventor, existing air-purifiers are associated with problems relating to keeping the filter of the air-purifier clean. As a result, filters typically need to be exchanged or at least be removed for cleaning the filter. Also, automatic cleaning mechanisms are not very efficient.

Therefore, in accordance with the present invention, an air purifier provided with filter cleaning that can provide a cleaning of the filter without a need to remove the filter is obtained. The filter cleaning can be made automatic and cleaned particles can be collected in a safe manner.

In accordance with a first aspect of the invention an air purifier with a filter cleaner for cleaning and sterilizing an air-filter is provided. The air filter can advantageously be placed in its operational position during the cleaning. The filter cleaner is configured to perform mechanical cleaning of the air-filter by making a cleaning member contact the surface of the air-filter for a first period of time, and to perform sterilization of the air-filter by applying UV light on the air-filter for a second period of time. Hereby an efficient cleaning of an air-filter can be achieved that removes dust and kills bacteria on the air-filter. The air-filter does not need to be removed during the cleaning and sterilization process.

In accordance with one embodiment, the cleaning member is located in a filter cleaning module. Hereby all parts used for cleaning the filter can be located in a single module that is easy to assemble or replace. Also, in accordance with one embodiment, at least one UV source can be located in the filter cleaning module.

In accordance with one embodiment, the filter cleaner is configured to activate a fan during air-filter cleaning. Hereby cleaning and also sterilization can be improved when cleaning the air-filter.

In accordance with one embodiment, the second period of time is longer than the first period of time. Hereby an efficient timing for the mechanical cleaning and sterilization procedure is obtained and the combined cleaning procedure is optimized to be efficient and at the same time require a small amount of time to perform.

### BRIEF DESCRIPTION OF THE DRAWINGS

The invention will now be described in more detail, by way of example, and with reference to the accompanying drawings, in which:
Fig. 1 is a view in perspective of an air purifier,
Fig. 2 is a top sectional view of an air purifier, and
Fig. 3 is an exploded view of a filter cleaner for an air purifier.
Fig. 4 is a flowchart illustrating steps performed when cleaning an- air filter.

### DETAILED DESCRIPTION

The invention will now be described more fully hereinafter with reference to the accompanying drawings, in which certain embodiments of the invention are shown. The invention may, however, be embodied in many different forms and should not be construed as limited to the embodiments set forth herein; rather, these embodiments are provided by way of example so that this disclosure will be thorough and complete, and will fully convey the scope of the invention to those skilled in the art. For example, like or similar components of different embodiments can be exchanged between different embodiments. For example, the filter unit as described herein is shown in a tower type air-purifier, but the filter unit could equally be mounted on any type of air purifier. Also, the filter cleaner is described to comprise a cleaning member that is configured to clean a rotating cylindrical filter located inside the air-purifier. However, other types of filters could also be used. Some components can be omitted from different embodiments. Like numbers refer to like elements throughout the description.

As has been realized existing air purifiers suffer from the drawback that the filter of the air purifier needs to be removed for efficient cleaning or replacement.

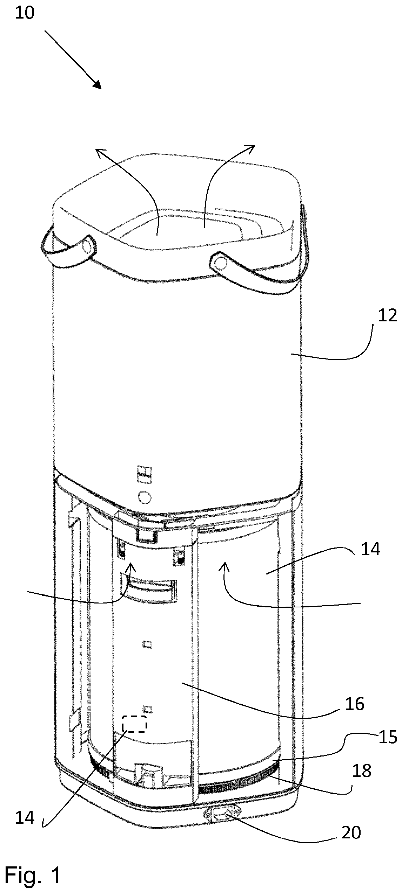

In Figs. 1-3 an air-purifier is described in which the air-filter cleaning in accordance with the invention can be used. However, it is to be noted that the invention is not limited to the embodiments of Figs 1-3. In Fig. 1 an air purifier 10 is depicted. In the exemplary embodiment shown in Fig. 1, the air-purifier 10 is of a tower type, i.e. a standing air-purifier having a bottom section configured to stand on a floor. A tower type of air-purifier is typically also elongated in an upwards direction when in use. However, the invention is not limited to such a type of air purifier. In an air-purifier of tower type air can be drawn into the air-purifier from the sides of the air-purifier by a fan. The air is made to pass an air-filter inside the air-purifier and the air the exits the air-purifier at the top of the air-purifier. In Fig. 1, the air-flow is generally indicated by the arrows.

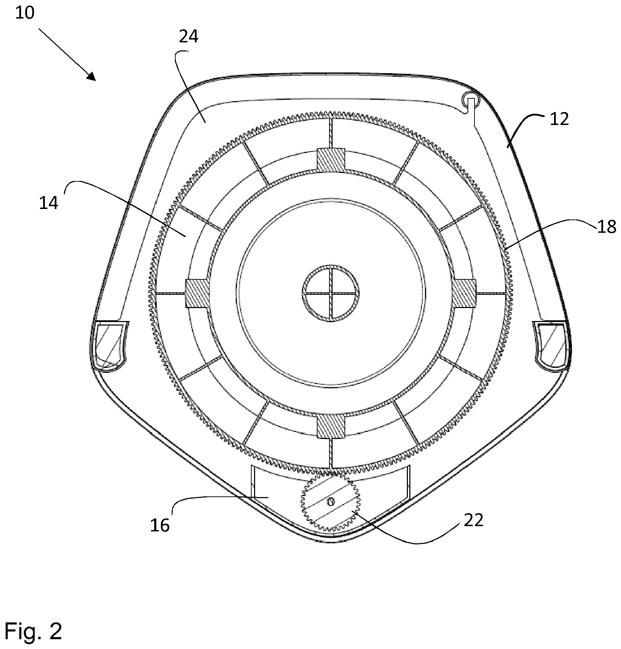

The air-purifier 10 has a casing 12. The casing 12 has a polygonal cross section as seen from the top of the air-purifier. The polygonal shape can be a regular polygonal shape. In particular the polygon can have at least five sides. The casing 12 shown in Fig. 1 is shaped as a regular polygon with five sides. Inside the casing a cylindrical filter 14 is provided. The cylindrical filter 14 can rotate inside the casing. In particular the cylindrical filter 14 can be made to rotate passed a filter cleaner. The cylindrical filter 14 can be placed in a filter holding case 15. The filter cleaner is here provided in a filter cleaner module 16. However, it is also envisaged that some parts of a filter cleaner are located outside the filter cleaner module. To drive the cylindrical filter a motor can be provided that via a gearing mechanism rotates the filter passed the filter cleaner module 16. To engage the rearing mechanism the cylindric filter can be provided with teeth 18. The filter cleaner module 16 can be located in a space formed between the outwards facing surface of the cylindric filter and a corner of the polygonal casing. This is better shown in Fig. 2. To supply power to the air-purifier a power connection 20 is provided. To control the cleaning of the filter cleaner a controller 17 can be provided. The controller 17 can comprise electric circuitry, a processor and a memory. The controller 17 can be configured to execute computer program instructions to control functions within the air-purifier 10. In particular, the controller 17 can be configured to perform a cleaning of an air-filter, such as an air-filter 14 of the air-purifier 10.

In Fig. 2, a top sectional view of the air-purifier 10 is shown. As can be seen in Fig. 2, there is a space 24 formed between the outer surface of the air-filter 14 and the polygonal casing 12 at the corners of the polygonal casing. At least one of such spaces 24 can be used to house various components of the air-purifier on a compact manner. In Fig. 2 a filter cleaner module 16 is provided in such a space. The filter cleaner can be provided in many different forms. In the embodiments shown herein, the filter cleaner is provided in a filter cleaner module housing many different components. This is advantageous for facilitating assembly and also for replacement. However, it is also envisaged that different components can be provided without being located in a filter cleaning module.

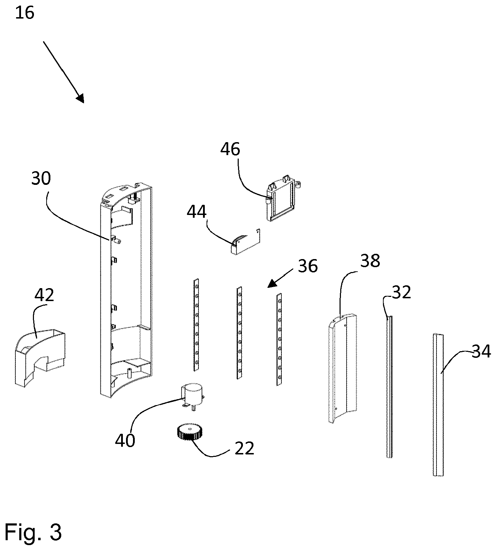

In Fig. 3, an exploded view of a filter cleaning module 16 is shown. As stated above some or all of the components can be provided separately and need not be part of the filter cleaning module 16. such a filter cleaner housed in a module is shown in an exploded view.

The filter cleaning module 16 has a housing 30. Inside the housing 30 a number of components can be located. In the exemplary embodiment shown in Fig. 3, cleaning members formed by a lamella 32 to scrape dust from an air-filter to be cleaned and a brush 34 are provided. Other types of cleaning members could also be envisaged in other embodiments such as any type of protruding structure adapted to contact a filter passing the cleaning member to remove dust and other particles from the filter.

Also, a UV light source such as at least one UV lamp 36 can be provided in the filter cleaning module 16 to clean the rotating filter from microorganisms such as bacteria and the like by killing it with the UV light. The UV light can be made to pass a lens 38 to make the light more efficient for eliminating micro-organisms. To rotate the filter in the air purifier, a motor 40 can be provided together with a gear mechanism 22. When the motor is run, the air-filter can be made to rotate via the gear mechanism being in connection with the air-filter, for example via teeth provided on the air filter. Further, a dust collection box 42 can be provided in the filter cleaning module. In accordance with one embodiment, the cleaning module is oriented in a generally vertical position with the dust collection box located at the bottom section. Thus, the cleaning members will then also be in a vertical position so that any material removed from the air filter will fall downwards. Any material removed from the filter will then be collected in the dust collection box 42 located below the cleaning members 32, 34. The components of the filter cleaning module 16 can be held in place by locking members 44, 46.

The filter cleaning module can be accessed via a door in the casing of the air-purifier. Further, for easy access of the dust collection box 42, the dust collection box can be removed backwards, radially outwards from the filter cleaning module. In this manner the dust collection box 42 can be easily pulled out from the filter cleaning module and emptied. To further facilitate emptying of the dust collection box, an indicator can be provided to indicate, for example, by a visual indication (not shown) that it is time to empty the dust collection box 42. The dust collection box 42 can be designed with a hollow mid-section for easy pulling with a finger. Also, the bottom surface of the dust collection box can have a sloping surface to collect the dust better.

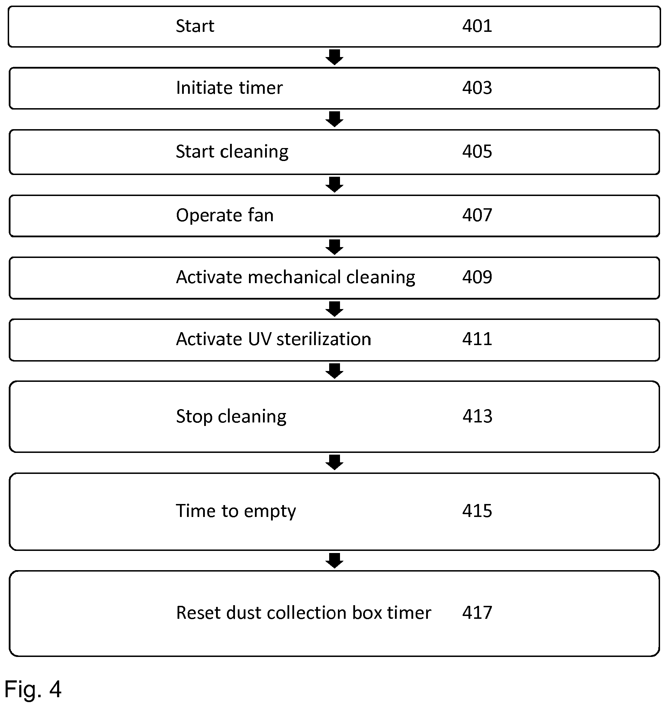

In Fig. 4 a flowchart illustrating steps that can be performed by a controller to clean an air filter. For ease of understanding it is here assumed that the air filter is the air filter described in figs. 1-3. However, it is to be understood that the cleaning mechanism for cleaning the air-filter can be applied to any type of air filter and is not restricted to a cleaning mechanism of the type of air filter described above. It is also to be noted that the flowchart of Fig. 4 is provided only as an example and that some steps can be omitted or supplemented. Steps can also be added to the cleaning procedure illustrated by Fig. 4.

First, in a step 401, the cleaning procedure is started. This can for example be initiated by turning the power for an air purifier on. This will in turn start the cleaning procedure of the air filter of the air purifier. Next, in a step 403, a timer is initiated. The timer can be configured to count the run time of the air-purifier or some other relevant parameter such as the time air is drawn through the air-filter. Then, in a step 405, when some predetermined condition is met the cleaning of the air-filter starts. The condition can for example be that the air -purifier has run for some pre-set time such as 12 h. Other conditions could also be used such as when some amount of air has been cleaned by the air-filter or when an air-fan has been on for some period of time. Next, in a step 407, a fan of the air-purifier can be activated. The fan can be run at a low speed. For example, the lowest speed setting of the fan of the air-purifier can be used. In another embodiment the fan speed is set to a speed below a pre-determined maximum speed. The fan is activated to improve dust collection and sterilization to be performed by blowing air on the air-filter during the mechanical cleaning and possibly also during the sterilization with UV light. Then, in a step 409, mechanical cleaning of the air-filter is activated. For example, a brush or some other cleaning member will be activated to remove dust from the surface of the filter. For example, in the air-purifier described above, the brush or lamella will be placed in a position such that when the filter is rotated passed to brush or lamella, the air -filter surface will contact the brush or lamella and dust will be scraped off from the air-filter surface and can be collected. The mechanical cleaning can be activated for a first period of time. For example, the mechanical cleaning can be activated for about 15 - 60 seconds or around 30 seconds. Hereby, there is time to scrape off dust from the entire air-filter surface, and at the same time the cleaning is performed at a relatively short time without risking to damage the air-filter.

Then, in a step 411, Ultra Violet (UV) light sterilization is performed for a second period of time by activating at least one UV lamp. The UV light can be lit for a longer time than the mechanical cleaning is performed. Thus, the second period of time can be longer than the first period of time. The second period of time can be about 30 - 120 seconds or around 60 seconds. Then, in a step 413, the cleaning is stopped and the air purifier can return to a normal mode of operation. The cleaning process can take about 60 - 180 seconds to perform. This will ensure a cleaned filter and the interruption of air purification will be very short. The dust removed from the mechanical filter cleaning is preferably removed from an air-purifier or any similar device in which the air-filter cleaning procedure is employed. Thus, in a step 415, after a third period of time the dust is to be removed. This can be signaled to a user via some light signaling audio signaling or a message being send to a mobile device or in some other suitable manner. The condition for when dust is to be removed can for example be a third period of time or a dust level sensor being placed in a dust collector that indicates when the dust collector needs to be emptied. In an embodiment where a timer is used to determine when to remove the dust, the time can be set to about two-weeks of operation of the device in which the air-filter is provided. Then, in a step 417, the dust is removed from the device. For example, in the device described above, a user can open the back door of the air-purifier and take out the dust collection box.

The dust collection box can be emptied and placed back in the air-purifier. A timer for emptying the dust collection box can be reset.

## Claims

1. An air purifier comprising :
- a casing (12) and cylindrical filter (14), wherein the casing (12) of the air purifier is polygonal such that a space (24) is formed between a corner of the polygonal casing and the cylindrical filter, the air purifier further comprising
- a filter cleaner module (16) located in said space (24) for cleaning and sterilizing an air-filter, the filter cleaner module being configured to:
- perform mechanical cleaning of the air-filter by making a cleaning member contact the surface of the air-filter for a first period of time, and then
- perform sterilization of the air-filter by applying UV light from at least one UV light source (36) located in the filter cleaning module (16).
on the air-filter for a second period of time.

2. The air-purifier according to claim 1, wherein the filter cleaner module is configured to activate a fan during air-filter cleaning.

3. The air-purifier according to any one of claims 1-2, wherein the second period of time is longer than the first period of time.

4. The air-purifier according to claim 3, wherein the first period of time is 15 - 60 seconds and the second period of time is 30 - 120 seconds.

5. The air-purifier according to any one of claims 1-4, wherein the filter cleaner module is configured to perform the filter cleaning and sterilization of an air-filter when the air-filter is positioned in a position to perform air filtering.

6. The air-purifier according to any one of claims 1-5, wherein a dust collection box (42) is provided in the filter cleaning module (16).

7. The air-purifier according to claim 6, wherein the dust collection box is located at the bottom section of the filter cleaning module (16).

8. The air-purifier according to claim 6 or 7, wherein the dust collection box is removable backwards, radially outwards from the filter cleaning module (16).

9. The air-purifier according to claim any one of claims 6-8, further comprising an indicator to indicate that it is time to empty the dust collection box (42).

## Patentansprüche

1. Luftreiniger, Folgendes umfassend:
- ein Gehäuse (12) und einen zylindrischen Filter (14), wobei das Gehäuse (12) des Luftreinigers vieleckig ist, so dass zwischen einer Ecke des vieleckigen Gehäuses und dem zylindrischen Filter ein Raum (24) gebildet ist, wobei der Luftreiniger ferner Folgendes umfasst:
- ein Filterreinigermodul (16), das sich in dem Raum (24) befindet, zum Reinigen und Sterilisieren eines Luftfilters, wobei das Filterreinigermodul für Folgendes gestaltet ist:
- Durchführen einer mechanischen Reinigung des Luftfilters durch In-Kontakt-Bringen eines Reinigungselements mit der Oberfläche des Luftfilters für eine erste Zeitspanne und dann
- Durchführen einer Sterilisation des Luftfilters durch Anwenden von UV-Licht von mindestens einer UV-Lichtquelle (36), die sich in dem Filterreinigermodul (16) befindet, auf den Luftfilter für eine zweite Zeitspanne.

2. Luftreiniger nach Anspruch 1, wobei das Filterreinigermodul dafür gestaltet ist, während der Luftfilterreinigung ein Gebläse zu aktivieren.

3. Luftreiniger nach einem der Ansprüche 1 bis 2, wobei die zweite Zeitspanne länger als die erste Zeitspanne ist.

4. Luftreiniger nach Anspruch 3, wobei die erste Zeitspanne 15 bis 60 Sekunden beträgt und die zweite Zeitspanne 30 bis 120 Sekunden beträgt.

5. Luftreiniger nach einem der Ansprüche 1 bis 4, wobei das Filterreinigermodul dafür gestaltet ist, die Filterreinigung und -sterilisation eines Luftfilters durchzuführen, wenn der Luftfilter in einer Position zum Durchführen der Luftfilterung positioniert ist.

6. Luftreiniger nach einem der Ansprüche 1 bis 5, wobei in dem Filterreinigermodul (16) ein Schmutzsammelkasten (42) bereitgestellt ist.

7. Luftreiniger nach Anspruch 6, wobei sich der Schmutzsammelkasten am Bodenabschnitt des Filterreinigermoduls (16) befindet.

8. Luftreiniger nach Anspruch 6 oder 7, wobei der Schmutzsammelkasten nach hinten, radial auswärts aus dem Filterreinigermodul (16) herausnehmbar ist.

9. Luftreiniger nach einem der Ansprüche 6 bis 8, ferner einen Indikator umfassend, um anzugeben, dass es an der Zeit ist, den Schmutzsammelkasten (42) zu leeren.

## Revendications

1. Purificateur d'air comprenant :
- un boîtier (12) et un filtre cylindrique (14), le boîtier (12) du purificateur d'air étant polygonal de sorte qu'un espace (24) est formé entre un coin du boîtier polygonal et le filtre cylindrique, ce purificateur d'air comprenant en outre :
- un module de nettoyage de filtre (16) situé dans ledit espace (24) pour nettoyer et stériliser un filtre à air, ce module de nettoyage de filtre étant configuré de façon à :
- exécuter un nettoyage mécanique du filtre à air en faisant en sorte qu'un élément de nettoyage entre en contact avec la surface du filtre à air pendant une première période de temps, puis à
- exécuter une stérilisation du filtre à air en appliquant une lumière UV venant d'au moins une source de lumière UV (36) située dans le module de nettoyage de filtre (16) sur le filtre à air pendant une deuxième période de temps.

2. Purificateur d'air selon la revendication 1, dans lequel le module de nettoyage de filtre est configuré de façon à activer un ventilateur pendant le nettoyage du filtre à air.

3. Purificateur d'air selon l'une quelconque des revendications 1 à 2, dans lequel la deuxième période de temps est plus longue que la première période de temps.

4. Purificateur d'air selon la revendication 3, dans lequel la première période de temps est 15 à 60 secondes et la deuxième période de temps est 30 à 120 secondes.

5. Purificateur d'air selon l'une quelconque des revendications 1 à 4, dans lequel le module de nettoyage de filtre est configuré de façon à exécuter le nettoyage de filtre et la stérilisation d'un filtre à air lorsque le filtre à air est positionné dans une position pour exécuter un filtrage de l'air.

6. Purificateur d'air selon l'une quelconque des revendications 1 à 5, dans lequel une boîte de collecte de poussière (42) est prévue dans le module de nettoyage de filtre (16).

7. Purificateur d'air selon la revendication 6, dans lequel la boîte de collecte de poussière est située au niveau de la section inférieure du module de nettoyage de filtre (16).

8. Purificateur d'air selon la revendication 6 ou 7, dans lequel la boîte de collecte de poussière peut être enlevée vers l'arrière, radialement vers l'extérieur depuis le module de nettoyage de filtre (16).

9. Purificateur d'air selon l'une quelconque des revendications 6 à 8, comprenant en outre un indicateur pour indiquer que le temps est venu de vider la boîte de collecte de poussière (42).
